# EUROPEAN PATENT APPLICATION

(11) **EP 1 270 733 A1**
(43) Date of publication of application: **02.01.2003**
(21) Application number: 01115080.2
(22) Date of filing: 21.06.2001
(51) Int. Cl.: C12N 15/87, C12N 15/10, A61K 47/02

(54) **Method for introducing a nucleic acid into a eukaryotic cell**

(71) Applicant: ECOLE POLYTECHNIQUE FEDERALE DE LAUSANNE, 1015 Lausanne (CH)
(72) Inventor: Girard, Philippe, 2540 Grenchen (CH); Jordan, Martin, 1024 Ecublens (CH); Wurm, Florian, 1025 St. Sulpice (CH)
(74) Representative: Kovacs, Paul

(57) **Abstract**

Described is method for introducing a desired nucleic acid into a eukaryotic host cell, said method comprising
a) admixing the desired nucleic acid, a mono- and/or divalent metal cation and a eukaryotic host cell lacking a cell wall
b) incubating the mixture to allow the eukaryotic host cell to take up the nucleic acid.

## Description

The ability to introduce foreign DNA into eukaryotic host cells is one of the principal tools of recombinant DNA technology. Methods for transfecting eukaryotic host cells with foreign DNA can be broadly grouped into four, categories: (1) direct introduction of cloned DNA by microinjection or microparticle bombardment; (2) use of viral vectors; (3) encapsulation within a carrier system; and (4) use of transfecting reagents such as calcium phosphate and diethylaminoethyl (DEAE) -dextran. Of the reagents used as facilitators of DNA transfection, calcium phosphate (CaPi) remains the most widely used because of its simplicity and general effectiveness for a wide variety of cell types.

A widely used protocol for CaPi/DNA transfection of mammalian cells (Jordan et al., Nucleic Acids Res. 24(4), 1996, 596-601) involves the formation of precipitates of CaPi and the DNA at standard concentrations of Ca²⁺, phosphate, and DNA, which are incubated for 1 to 20 minutes at room temperature before being added to the host cell in medium. The cells are exposed 2 to 6 hours to the precipitate before medium is replaced by fresh medium. A variation of the calcium phosphate method for introducing DNA into a eukaryotic host cells has been described in WO 00/39320. The process described therein comprises the preparation of a precipitate of calcium phosphate and DNA by admixing 125-250 mM Ca²⁺, 0.2-0.5 mM phosphate and 25-100 µg/ml DNA, incubating the precipitation mixture for about 1 to 40 minutes to form a precipitation mixture which is further diluted and admixed with the eukaryotic cell to form a transfection mixture which is incubated for uptake of the precipitate into the cell and then incubated with fresh medium.

The methods described above have some drawbacks that are experienced when using the methods. The length of time required for the formation of precipitates of CaPi and the DNA is critical to the efficiency. If the time required for the formation of precipitates of CaPi and the DNA is less than required or exceeds a defined time frame, transfection will become less efficient. Furthermore, the time frame needed for the formation of precipitates of CaPi and the DNA has to be optimised for other reactants such as the DNA and the phosphate concentration used. This is particular disadvantageous if the process is scaled up e.g. to large volumes of suspension cultures wherein the approriate admixing is difficult to achieve. Furthermore, in methods applying highthroughput techniques for transfecting a large number of individual cell cultures time constraints are not desirable. In addition, the formation of optimal precipitates of CaPi and the DNA is dependent on a narrow range of physical, chemical and dynamic parameters. The narrow time constraints for the formation and use of optimal precipitates of CaPi and the DNA prevent the long-term storage of the precipitates of CaPi and the DNA prior to transfection.
Both methods require several steps such as the formation of a precipitation mixture, a dilution step for the formation of a transfection mixture which is further incubated for uptake of the nucleic acid into the cell. Such a multi-step process is time consuming and costly.

The object of the present invention is to provide an improved method which does not have the above-mentioned disadvantages and which can easily be applied for introducing a desired nucleic acid into a eukaryotic host cell.
This object has been achieved with a novel method according to claim 1.
It has been surprisingly found that, according to the invention, a desired nucleic acid can be introduced into a eukaryotic host cell by a method comprising
a) admixing the desired nucleic acid, a mono- and/or divalent metal cation and a eukaryotic host cell lacking a cell wall
b) incubating the mixture to allow the eukaryotic host cell to take up the nucleic acid.

**Figure 1** shows the efficiency of transfection measured as expression of GFP using different metal cations compared to calcium phosphate transfection (CaPi).

**Figure 2** shows the efficiency of transfection measured as expression of GFP under different conditions.

**Figure 3** shows the efficiency of transfection measured as expression of GFP. HEK 293 EBNA cells were grown in suspension and transfected in suspension and then left to adhere.

**Figure 4** shows the efficiency of transfection measured as expression of human anti RhD-antibody. HEK 293 EBNA cells were grown in suspension and transfected in suspension and then left to adhere.

**Figure 5** shows the efficiency of transfection measured as expression of GFP. HEK 293 EBNA cells were grown in adherence and transfected in suspension and then left to adhere.

**Figure 6** shows the efficiency of transfection measured as expression of human anti RhD-antibody. HEK 293 EBNA cells were grown in adherence and transfected in suspension and then left to adhere.

**Figure 7** shows the impact of different conditions on storage of a DNA/ Ca²⁺ solution.

### Desired nucleic acid

The terms "transfection", "introducing a desired nucleic acid into a eukaryotic host cell", "uptake of nucleic acid by a host cell", and "taking up of nucleic acid by a host cell", denote any process wherein a nucleic acid, with or without accompanying material, enters a host cell. The term "cell transfected" or "transfected cell" means the cell into which the nucleic acid has been introduced and thus harbours the nucleic acid. The nucleic acid might be introduced into the cell so that the nucleic acid is replicable either as a chromosomal integrant or as an extrachromosomal element.

As used herein, the term "desired nucleic acid" refers to any desired DNA, RNA or DNA/RNA hybrid, including those contained on a vector e. g. an expression vector such as a plasmid or an infectious vector such as a retroviral, herpes, adenovirus, adenovirus-associated, mumps and poliovirus vector.
DNA which can be used in the present invention is any polydeoxynucleotide, including, e.g., double-stranded DNA, single- stranded DNA, double-stranded DNA wherein one or both strands are composed of two or more fragments, double-stranded DNA wherein one or both strands have an uninterrupted phosphodiester backbone, DNA containing one or more single-stranded portion(s) and one or more double-stranded portion(s), double-stranded DNA wherein the DNA strands are fully complementary, double-stranded DNA wherein the DNA strands are only partially complementary, circular DNA, covalently- closed DNA, linear DNA, covalently cross-linked DNA, cDNA, chemically- synthesized DNA, semi-synthetic DNA, biosynthetic DNA, naturally-isolated DNA, enzyme-digested DNA, sheared DNA, plasmid DNA, chromosomal DNA, labeled DNA, such as radiolabeled DNA and fluorochrome-labeled DNA, DNA containing one or more non-naturally occurring species of nucleic acid.

RNA which can be used in the present invention is any polyribonucleotide, including, e.g., single-stranded RNA, double- stranded RNA, double-stranded RNA wherein one or both strands are composed of two or more fragments, double-stranded RNA wherein one or both strands have an uninterrupted phosphodiester backbone, RNA containing one or more single-stranded portion(s) and one or more double-stranded portion(s), double-stranded RNA wherein the RNA strands are fully complementary, double-stranded RNA wherein the RNA strands are only partially complementary, covalentlycrosslinked RNA, enzyme-digested RNA, sheared RNA, mRNA, hnRNA, tRNA, including both charged and uncharged tRNA, rRNA, all forms of viral genomic RNA, chemically-synthesized RNA, semi-synthetic RNA, biosynthetic RNA, naturally-isolated RNA, labeled RNA, such as radiolabeled RNA and fluorochrome-labeled RNA, RNA containing one or more non-naturally- occurring species of nucleic acid.

DNA/RNA hybrid which can used is any hybrid nucleic acid comprising one strand of DNA and one strand of RNA wherein the DNA strand and the RNA strand form a species that is at least partially double-stranded, including hybrids wherein the DNA strand is fully complementary or only partially complementary to the RNA strand, hybrids wherein the DNA strand and/or the RNA strand has (have) an uninterrupted phosphodiester backbone, hybrids wherein the DNA strand and/or the RNA strand is (are) composed of two or more fragments, hybrids containing one or more single-stranded portion(s) and one or more double-stranded portion(s), hybrids created by reverse transcription of RNA, hybrids created by transcription of DNA, hybrids created by annealing of complementary or partially-complementary DNA and RNA, covalently cross-linked hybrids, chemically-synthesized hybrids, semi-synthetic hybrids, biosynthetic hybrids, naturally-isolated hybrids, labeled hybrids, such as radiolabeled hybrids and fluorochrome-labeled hybrids, hybrids containing one or more non-naturally occurring species of nucleic acid. Preferably DNA, more preferably double-stranded DNA, most preferably plasmid DNA is used in the present invention.

The DNA for use in the methods of the invention can be prepared by a variety of methods known in the art. These methods include, but are not limited to, chemical synthesis e. g. by the triester, phosphite, phosphoramidite, and H-phosphonate methods as desribed e. g. in Engels *et al*., Angew. Chem. Int. Ed. Engl., 28. 716-734 (1989). Alternatively, the desired DNA sequences can be obtained from existing clones or, if none are available, by screening DNA libraries and constructing the desired DNA sequences from the library clones. Suitable quantities of DNA for use herein can be produced by amplifying the DNA in well-known cloning vectors and hosts, such as plasmid vectors carrying the pBR322 origin of replication for autonomous replication in most Gram-negative bacterial hosts such as E. coli, plasmid vectors carrying the pC194 origin of replication for autonomous replication in *Bacillus* and some other Gram-positive bacterial hosts, or 2-micron circle (2µ plasmid) vectors carrying an origin of replication for autonomous replication in most yeast hosts. Alternatively, the DNA template can be amplified by polymerase chain reaction (PCR) as described e. g. by Saiki et al., Science, 230, 1350 (1985). Preferably, the DNA for use in the methods of the invention is prepared by amplifying the DNA in a plasmid vector in E. coli as host.

The RNA for use in the methods of the invention can be prepared by a variety of methods known in the art. These methods include, but are not limited to, chemical synthesis of RNA, and *in vitro* translation of a DNA template as described generally in Current Protocols in Molecular Biology (Wiley Interscience: New York, 1990). Alternatively, the desired RNA can be isolated from total cellular RNA extracted from a host cell culture. Total cellular RNA can be isolated from the host cell culture by any method known in the art such as, in the case of RNA produced in mammalian host cells, the methods described by e. g. Favaloro et al., Methods Enzymol 65, 718 (1980). If the desired RNA is a polyadenylated mRNA fraction of total cellular RNA, the polyadenylated mRNA can be separated from the bulk of cellular RNA by affinity chromatography on oligodeoxythymidylate (oligo (dT) ) -cellulose columns using any method known in the art, such as the method of Edmonds et *al*., Proc. Natl. Acad, Sci., USA, 68: 1336 (1971).
In addition, the desired RNA can be obtained from the recombinant or non-recombinant genome of an RNA virus, including single-stranded RNA viruses, such as retroviruses, tobacco mosaic viruses, influenza viruses, Newcastle disease virus, and double-stranded RNA viruses such as rotaviruses and rice dwarf virus. The desired RNA can be isolated by growing up the chosen RNA virus in a suitable host cell culture, harvesting the viral particles, and then extracting the desired RNA from the viral particles.

The DNA/RNA hybrids suitable for use in the methods of the invention can be prepared by any method known in the art. In one embodiment, the DNA strand or DNA fragments are produced as described above, the RNA strand or fragments are produced as described above, and the DNA and RNA strands or fragments are admixed together and allowed to anneal. In another embodiment, the DNA/RNA hybrid can be produced by obtaining the desired DNA strand as described above, using the DNA strand as a template to drive synthesis of the complementary RNA strand by a DNA-directed RNA polymerase, and harvesting the DNA/RNA hybrid upon completion of the transcription reaction. Alternatively, the DNA/RNA hybrid can be prepared by obtaining the desired RNA strand as described above, using the RNA strand as a template to drive synthesis of the complementary DNA strand by a RNA-directed DNA polymerase, and harvesting the DNA/RNA hybrid upon completion of the reverse- transcription reaction.

The nucleic acid introduced into the eukaryotic host cell lacking a cell wall by the method of the present invention might comprise a fragment encoding a polypeptide and may include additional regulatory sequences as known in the art e.g. a promoter and/or an enhancer, polyadenylation sites and splice junctions usually employed for the expression of the polypeptide or may include additionally one or more separate targeting sequences and may optionally encode a selectable marker. Promoters which can be used provided that such promoters are compatible with the host cell are e.g promoters obtained from the genomes of viruses such as polyoma virus, adenovirus (such as Adenovirus 2), papilloma virus (such as bovine papilloma virus), avian sarcoma virus, cytomegalovirus (such as murine or human cytomegalovirus immediate early promoter), a retrovirus, hepatitis-B virus, and Simian Virus 40 (such as SV 40 early and late promoters) or promoters obtained from heterologous mammalian promoters, such as the actin promoter or an immunoglobulin promoter or heat shock promoters. Enhancers which can be used are e. g. enhancer sequences known from mammalian genes (globin, elastase, albumin, a-fetoprotein, and insulin) or enhancer from a eukaryotic cell virus. e. g. the SV40 enhancer, the cytomegalovirus early promoter enhancer, the polyoma, and adenovirus enhancers.
The nucleic acid introduced into the eukaryotic host cell lacking a cell wall might also comprise non-polypeptide encoding gene fragments or other transcribed nucleic acid sequences as well as non-transcribed nucleic acid with biological function, when introduced into the cell such as e. g. ubiquitous chromatin opening elements (UCOE) as described in (Benton et al. Abstracts ESACT meeting, Tylösand Sweden, June 10-14 2001). As used herein "polypeptide" refers generally to peptides and proteins having more than about ten amino acids. The polypeptides may be "homologous" to the host (i.e., endogenous to the host cell being utilized), or "heterologous," (i.e., foreign to the host cell being utilized), such as a human protein produced by yeast. The polypeptide may be produced as an insoluble aggregate or as a soluble polypeptide in the periplasmic space or cytoplasm of the cell, or in the extracellular medium.
Examples of polypeptides include hormones such as growth hormone, growth factors such as epidermal growth factor, antibodies such as IgG antibodies, and receptors to hormones or growth factors and includes as well polypeptides usually used as a visualising marker e.g. green fluorescent protein. The preferred polypeptide herein is an antibody, more preferably an IgG antibody. Depending on whether the nucleic acid nucleic acid is integrated as a chromosomal integrant or remains an extrachromosomal element the polypeptide is stably or transiently expressed.

Depending on the mode of preparation of the nucleic acid before introducing the nucleic acid into the eukaryotic host cell, the nucleic acid might be purified according to methods known in the art e. g, caesium chloride gradient centrifugation, gel electrophoresis or ion exchange chromatography.
The nucleic acid can be suspended in buffer, such as TE(Tris/EDTA)-buffer prior to being admixed to the mono and/or divalent ion and the host cell. The nucleic acid is usually admixed to the mono and/or divalent ion and the host cell so that the concentration of the nucleic acid in the mixture is 0.1 to 10000 µg/ml, preferably 0.2 to 500 µg/ml, more preferably 0.5 to 50 µg/ml, most preferably 1 to 5 µg/ml.

### Mono and/or divalent metal cation

As mono and/or divalent metal cation e. g. Li⁺, Na⁺, K⁺, Cs⁺, Ca²⁺, Ba²⁺ and/or Sr²⁺ can be used in the present invention. Preferably, divalent metal cations such as Ca²⁺, Ba²⁺ and/or Sr²⁺, more preferably Ca²⁺ or Ba²⁺, most preferably Ca²⁺ is used. The mono and/or divalent metal cation is usually prepared prior to admixture to the nucleic acid and the host cell by dissolving the appropriate salt such as e. g. the chloride, carbonate or fluoride salt of the metal in water, preferably deionised water. In case the mono and/or divalent metal cation is prepared prior to admixture to the nucleic acid and the host cell by dissolving the appropriate salt, usually no phosphate salt of the metal is used. The concentration of the metal cation dissolved in water is usually 10 mM to 10 M, preferably 50 mM to 1 M, more preferably 100 mM to 500 mM.
The mono and/or divalent metal cation is usually admixed to the nucleic acid and the host cell so that the concentration of the mono and/or divalent metal cation in the mixture is 0.001 mM to 2 M, preferably 1 mM to 200 mM, more preferably 8 mM to 15 mM.

### Eukaryotic host cell

As used herein, the term "eukaryotic host cell lacking a cell wall" refers to any nucleated cell that has no cell wall in the cell's native state, including all vertebrate cells, such as mammalian cells, avian cells, reptilian cells, amphibian cells, and fish cells, cells of multicellular invertebrate animals, such as insect cells, crustacean cells, and mollusk cells, cells of protozoans, *etc.,* and to any nucleated cell that has had its native cell wall removed or is in a natural or artificially-induced state wherein no cell wall is present, including all plant cells that are capable of forming protoplasts or are capable of being treated to form protoplasts.

Any eukaryotic host cell lacking a cell wall can be used in the methods of the invention. Examples of useful mammalian host cell lines include human cells such as human embryonic kidney line (293 or 293 cells subcloned for growth in suspension culture, Graham et al., J. Gen Virol 36, 59 (1977), human cervical carcinoma cells (HELA, ATCC CCL 2), human lung cells (W138, ATCC CCL 75), human liver cells (Hep G2, HB 8065), human HKB11 cells (Cho et al. Abstracts ESACT meeting, Tylösand Sweden, June 10.14 2001); rodent cells such as baby hamster kidney cells (BHK, ATCC CCL 10), Chinese hamster ovary cells/-DHFR (CHO, Urlaub and Chasin, Proc. Natl. Acad. Sci. USA, 77, 4216 (1980)), mouse sertoli cells (TM4, Mather, Biol. Reprod 23, 243-251 (1980)), mouse mammary tumor (MMT 060562, ATCC CCL51); and cells from other mammals such as monkey kidney CV1 line transformed by SV4O (COS-7, ATCC CRL 1651); monkey kidney cells (CV1 ATCC CCL 70); African green monkey kidney cells (VERO-76, ATCC CRL-1587); canine kidney cells (MDCK, ATCC CCL 34); buffalo rat liver cells (BRL 3A, ATCC CRL 1442); NS0 cells (Kohler et al., Eur. J. Immunol. 6, 292); mouse 3T3 cells (ATCC CCL-92). Preferred for use herein are mammalian cells. More preferably human cells are used.

The mammalian host cell of choice can be cultured by any method known in art, such as, e.g., growing the cells adherently e. g. as a monolayer in T-Flasks or growing the cells in suspension culture e. g. in spinner flasks or in air lift or stirred tank bioreactors. Standard techniques, methods, and equipment are reviewed in Lubiniecki, ed., Large Scale Mammalian Cell Culture Technology (Marcel Dekker: New York and Basle, 1990).
In the case of plant cell hosts, the plant cell protoplast cultures suitable for use herein can be prepared according to the method of Lichtenstein and Draper, "Genetic Engineering of Plants", in DNA Cloning Volume III: A Practical Approach, Glover, ed, (IRL Press, 1985, pp.67-119).

### Mode of introduction of desired nucleic acid

The nucleic acid might be introduced into eukaryotic cells e. g. for delivery to eukaryotic target tissues in a drug-delivery or gene-therapy mode or might be introduced into eukaryotic cells cultured in an appropriate medium or cells kept in e. g. buffer solution such as N-3-hydroxyethylpiperazine-N'-3-ethanesulfonic acid (HEPES) . Preferably, the nucleic acid is introduced into eukaryotic cells cultured in an appropriate medium. As medium common basal medium supplemented with about 0.1 to 20 %, preferably 0.2 to 10 %, more preferably 0.5 to 2 % serum as well as serum-free, common basal medium can be used. Also, common basal medium free of protein of animal origin can be used. Preferably serum-containing common basal medium is used. Sera that may be used are e. g. foetal calf serum or adult bovine serum . Preferably, foetal calf serum is used.
Common basal medium that may be used depends on the eukaryotic host cell used. Common basal medium are e. g. Eagle's minimal essential medium (MEM) medium, Dulbecco's Modification of Eagle's Medium (DMEM), Iscove's DMEM medium (N. Iscove and F. Melchers, Journal of Experimental Methods, 1978, 147, 923), Ham's F12 medium (R.G. Ham, Proceedings of National Academy of Science, 1965, 53, 288), L-15 medium (A. Leibovitz, American Journal of Hygiene, 1963, 78, 173), RPMI 1640 medium (G.E. Morre et al., The Journal of the American Medical Association, 1967, 199, 519), Pro 293s CDM medium (BioWhittaker, Walkersville, MD) and suitable ratio mixtures thereof.
Common supplements might be added to the common basal medium. Supplements that might be usually added contain proteins usually present in serum and optionally further ingredients which have a positive effect on cell growth and/or cell viability. Proteins usually present in serum are e.g. bovine serum albumin (BSA), transferrin and/or insulin. Further ingredients which have a positive effect on cell growth and/or cell viability are e.g. soybean lipid, selenium and ethanolamine. Amino acids and/or nucleosides might be added to the culture medium depending on the cell line. Examples of amino acids are isoleucine, leucine, valine, lysine, asparagine, aspartic acid, glutamine, glutamic acid, proline, serine, alanine. Peptones of animal , plant or yeast origin might be added as well.

In case the cells have been cultured in the appropriate medium the nucleic acid and the mono- and/or divalent metal cation can be admixed directly to the cells after having grown in culture to a desired cell mass or the medium is removed from the cells after having grown in culture to a desired cell mass and the cells are exposed to newly added medium before admixing the cells in the medium with the nucleic acid and the mono- and/or divalent metal cation. Usually the medium is removed from the cells after having grown in culture to a desired cell mass and the cells are exposed to newly added medium before admixing the cells in the medium with the nucleic acid and the mono- and/or divalent metal cation. The newly added medium can be the same medium as used for growing the cells or might be a different common basal medium. In case the medium used for growing the cells contains components interacting negatively with transfection e. g. heparin or proteoglycan as described in Mounkes et al. (J. Biol. Chem. 273(40), 26164-70) the newly added medium is changed to a medium not containing such components. The newly added medium might contain serum as described above or might be serum-free. Preferably, the newly added medium contains serum. In case new medium has been added, the cells can be usually admixed directly with the nucleic acid and the mono- and/or divalent metal cation without adapting the cells first to the newly added medium. Preferably, the cells are exposed to the medium 0.1 to 24 hours, preferably 1 to 12 hours, more preferably 1.5 to 4 hours before admixing the cells with the nucleic acid and the mono- and/or divalent metal cation. Usually, suspended cells are admixed with the nucleic acid and the mono- and/or divalent metal cation Cells grown adherently are usually detached by e. g. adding trypsin prior being admixed with the nucleic acid and the mono- and/or divalent metal cation. Preferably, the mono- and/or divalent metal cation, the desired nucleic acid and the eukaryotic host cell lacking a cell wall are admixed in serum containing medium.

Usually, a cell mass of greater than about 1x10⁴ cells/ml liquid volume, preferably between 2x10⁴ and 10⁸ cells/ml liquid volume, more preferably between 10⁵ and 10⁷ cells/ml liquid volume, is used for adherent or suspended cells. The cell mass for transfection in suspension culture can be about 0.01% to about 5% packed cell volume (PCV). However, the invention also encompasses the use of higher or lower cell densities that provide acceptable levels of transfection in suspension culture. For example, the invention can be practiced by concentrating cells from a bioreactor to obtain a high-mass cell slurry, and then admixing the the nucleic acid and the mono- and/or divalent metal cation with the cell slurry.

The order of admixing the mono- and/or divalent metal cation, the desired nucleic acid and the eukaryotic host cell lacking a cell wall is not important for practicing the invention. The cells can be admixed with a solution containing the nucleic acid and the mono-and/or divalent metal cation or might be admixed first with the nucleic acid and then admixed with the missing mono-and/or divalent metal cation component or might be admixed first with the mono-and/or divalent metal cation and then admixed with the missing nucleic acid. Usually, the cells are admixed with a solution containing the nucleic acid and the mono-and/or divalent metal cation. In this case the solution containing the nucleic acid and the mono-and/or divalent metal cation might be stored under different conditions as indicated below and/or might be filtered before being admixed to the cells in order to sterilize the solution before being admixed to the cells.

The mixture containing the cells, the nucleic acid and the mono-and/or divalent metal cation is usually incubated for the time period needed to allow the eukaryotic host cell to take up the nucleic acid. Usually, the mixture containing the cells, the nucleic acid and the mono-and/or divalent metal cation is incubated for a time period of 0.5 to 24 hours, preferably 1 to 12 hours, more preferably 4 to 6 hours to allow the eukaryotic host cell to take up the nucleic acid. During the incubation the cells might be suspended or might be left to adhere. The temperature during incubation of the mixture is usually 20 to 50°C, preferably 35 to 40°C, most preferably 37°C. The pH of the mixture incubated is usually between 5 and 9, preferably 6.5 to 8, more preferably 7.4 to 7.8. The time to allow the eukaryotic host cell to take up the nucleic acid and the temperature of incubation, however, may vary with the particular cell used. A pH buffer that is effective at a pH range encompassing the desired pH for the mixture can be used to control the pH. Buffers that are suitable for use herein include appropriate concentrations of HEPES or HEPES -buffered saline. The buffer might be added to the medium in case the cells have been cultured, prior to be added to the mixture or might be added directly to the mixture. Admixture and incubation can take place e. g. in a flask or a bioreactor as described above in case the cells have been cultured prior to being admixed to the nucleic acid and the mono-and/or divalent metal cation or might occur during passage of the cells from one culture vessel to another. As evident to a person skilled in the art the method of the present invention can be applied in small scale e.g. in the range of nanoliters or microliters such as used in microchip or microtiter well applications and in large scale e. g. in suspension cultures of at least 10 liter, preferably 10 to 10000 liter.

Depending on the mode of introducing the nucleic acid into the cell, the cell and the amount of cells used transfection of usually at least 1%, preferably at least 10%, more preferably at least 30 %, most preferably at least 50 % of the cells incubated is achieved after the above indicated time period of incubation. In case the nucleic acid is introduced into eukaryotic cells cultured in an appropriate medium transfection of usually at least 50%, preferably at least 70%, more preferably at least 90 %, most preferably at least 95 % of the cells incubated is achieved after the above indicated time period of incubation.
Depending on their further use, the cells can be further incubated without further dilution of the mixture after the incubation incubated for another 200 hours, preferably 48-96 hours. Common supplements as indicated above can be added to the such further incubated cells in order to support growth of the cell. The cells can be as well separated from the mixture without further dilution of the mixture by e.g. seeding the cells, discarding the supernatant and resuspending the cells in fresh medium and can be cultured e.g. in the appropriate medium after the incubation.
Alternatively, the incubated mixture can be diluted after the incubation by the addition of medium and the diluted mixture can be incubated for another 300 hours, preferably 200 hours. Preferably, the cells are separated from the mixture after the incubation without further dilution of the mixture.
The rate of transfection is usually measured after the mixture has been incubated for the above indicated period of time period according to methods known in the art. The method of measuring the rate of transfection depends of the kind of nucleic acid introduced into the cell. In case the nucleic acid introduced into the cell is DNA encoding a polypeptide usually the expression of a polypeptide encoded by the DNA is measured by e.g ELISA or in case the protein is visual by e. g. fluorescence emission. In case the polypeptide is secreted from the cell into the mixture expression can be directly measured-by taking an appropriate sample from the mixture. In case the polypeptide is not secreted the cells have to be lysed before measuring the expression of the polypeptide.

A further aspect of the present invention is to provide a method for storing a a nucleic acid. The object has been achieved with a novel method according to claim 12.
According to the invention a nucleic acid is stored by a method comprising
a) admixing a mono- and/or divalent metal cation and a desired nucleic acid
b) storing the solution of mono- and/or divalent metal cation and a desired nucleic acid for at least 8 weeks.

As the mono- and/or divalent metal cation the above mentioned mono- and/or divalent metal cations can be used. Preferably a divalent metal cation such as Ca²⁺, Ba²⁺and/or Sr²⁺ are used, more preferably Ca²⁺or Ba²⁺, most preferably Ca²⁺ is used. The preparation of the mono- and/or divalent metal cation is as described above. The concentration of the metal cation in the solution of mono- and/or divalent metal cation and a desired nucleic acid is usually 10 mM to 10 M, preferably 50 mM to 1 M, more preferably 100 mM to 500 mM. As desired nucleic acid the above described nucleic acids can be used. Preferably, the nucleic acid is DNA and is prepared as described above.
The order of admixing the mono- and/or divalent metal cation and the desired nucleic acid is not important for practicing the method.
The solution can be stored for at least 8 weeks, preferably for at least 16 weeks, more preferably for at least 52 weeks. The solution can be stored at a temperature range from about room temperature, to about - 80°C without loss of transfection efficiency if used thereafter for introducing the nucleic acid into a eukaryotic host cell according to the method of the invention described above, whereby depending on the mode of introducing the nucleic acid into the cell, the cell and the amount of cells used transfection of usually at least 1%, preferably at least 10%, more preferably at least 30 %, most preferably at least 50 % of the cells incubated is achieved after the above indicated time period of incubation. In case the nucleic acid is introduced into eukaryotic cells cultured in an appropriate medium transfection of usually at least 50%, preferably at least 70%, more preferably at least 90 %, most preferably at least 95 % of the cells incubated is achieved after the above indicated time period of incubation.

### Examples

### 1. Preparation of nucleic acid

Plasmid DNA is amplified in the E.Coli strain DH5α (J. Mol. Biol. 1983, 166(4), 557-80, GibcoBRL, Cat.No. 18263-012) after a heat shock transformation of the bacteria (Sambrook et *al*., Molecular Cloning: A Laboratory Manual 2nd Ed. (Cold Spring Harbor Laboratory Press: Cold Spring Harbor, New York, 1989). The plasmid is purified through a silica-based ion exchange Machery-Nagel AX 10000, and DNA purity is controlled by OD 260/280 measurement, where a minimum ratio of 1.8 has been fixed. Two different plasmids encoding for Green fluorescent protein and encoding for the heavy and light chains of the human IgG antibody are used.
Green fluorescent protein DNA is contained in the pEGFP-N1 plasmid (Clontech, Palo Alto, CA, USA), and the heavy and light chains of the human IgG antibody, an Anti-RhD antibody, have been cloned into the pEAX8 backbone (EdgeBioSystems, Gaithersburg, MD) after cleavage at the two BstX I 2874 restriction sites and deletion of the Kil A region (pEAX39LH1 and pEAX41LH2). The lengths of the heavy and light chains are approximately 3 kbp.

E.coli DH5α transformed with the respective plasmid, are inoculated from a glycerol stock into 3ml pre cultures (LB medium) and grown for 10 h in an orbital shaker (INFORS Bottmingen, Switzerland) at 37°C and 130 rpm. These pre-cultures are then used to inoculate 1 l volumes of LB in 3 l erlenmeyer flasks, which are grown under the same conditions as the pre cultures for 14 hours. This gives a final optical density at 600 nm of around 3. Plasmid extraction and purification is performed using a standard kit protocol Nucleobond® AX (Macherey & Nagel GmbH & Co.KG Dürren, Germany) essentially a combination of alkaline lysis and anion exchange chromatography. All chemicals for DNA purification are from Macherey & Nagel if not mentioned otherwise. The steps of the procedure include:
*1. Cell disruption:*
   2 x 1l volumes of fermentation broth are harvested at 4,000 x g by centrifugation and resuspended in 80 ml buffer S1 (50 mM Tris/HCl, 10 mM EDTA, 100 µg Rnase A/ml, pH 8.0). The cells are lysed by the addition of 80 ml buffer S2 (0.2 M NaOH, 1% SDS) and mixed by inverting, the lysate is then incubated for 5 minutes at room temperature. Finally 80 ml buffer S3 (2.80 M KAc, pH 5.1) is added to precipitate cell debris and chromosomal DNA. The solid precipitate is separated from the plasmid containing supernatant by centrifugation (conditions as above).
*2. Filtration:*
   Supernatants is cleared over a folded filter and collected in a 50 ml plastic tube.
*3. Equilibration:*
   The Nucleobond® AX 10000 cartridge is equilibrated with 100 ml of buffer N2 (100 mM Tris, 15% EtOH and 900 mM KCl adjusted with H₃PO₄ to pH 6.3)
*4. Adsorption:*
   *The cleared supernatents are passed over the AX 10000 column*
*5. Wash:*
   The cartridge is washed with 2 times 100 ml of buffer N3 (100 mM Tris, 15% EtOH and 1000 mM KCl adjusted with H₃PO₄ to pH 8.5)
*6. Elution, Precipitation and Resuspension:*
   After elution with buffer N5 (100 mM Tris, 15% EtOH and 900 mM KCl adjusted with H₃PO₄ to pH 6.3) plasmid DNA is concentrated by isopropanol precipitation (0.6 volume isopropanol (Fluka Chemie AG Buchs Switzerland) to 1 volume eluate) and centrifuged at maximum speed. Then it is washed in 70% EtOH (Fluka) and centrifuged at 15000 x g for 10 mn. Finally it is dried and resuspended in 2 ml of TE buffer (pH 8.0).

### 2. Metal cation solutions

Metal cation solutions were prepared with ddH₂O dissolving the respective amounts of Chloride, Fluoride or Carbonate salt of the following metal cations: Li⁺, Na⁺, K⁺, Cs⁺, Ca²⁺, Sr²⁺, Ba²⁺. Concentrations of the metal cations ranged from 10 mM to 10 M for the DNA/metal cation complexing step. All salts came from Fluka puriss. p.a.

### 3. Host cell line

Transfection has been performed with the HEK 293 EBNA (Eptstein nuclear antigen) cell line derived from Graham et al., J. Gen Virol 36, 59 1977 (Invitrogen Cat. No R620-07), both suspension grown and adherent cells.
During normal cell culture, cells are incubated in a Roll-In incubator (Bellco Glass, Inc. Vineland, NJ) at 37°C in BioWhittaker's Pro 293s CDM medium (BioWhittaker, Inc.

Walkersville, MD), which is capable to sustain serum-free growth. The medium is supplemented with 4 mM L-glutamine and 0.02 mM phenol red. Cells are cultured at pH ranging from 6.7 to 7.6. Cells are passaged twice a week (medium change and dilution).

### 4. Transfection

Cells have been transfected in FEME medium, which is DMEM/ Ham's F12 medium supplemented with 10 mM HEPES (free acid), 5 g/l D-glucose, 2.5 mg/ml insulin, 2.5 mg/ml transferrin, 100 µM diethanolamine, 100 µM L-proline and 2.5 Mm L-glutamine (all from Sigma St. Louis, MO). If necessary, pH was adjusted to 7.1 - 7.2 with CO₂ (Carba Gas Bern, Switzerland). Transfections have been carried out with pH ranging from 6.5 to 8.0.

### A. Transfection measured as expression of GFP using different metal cations compared to calcium phosphate transfection (CaPi).

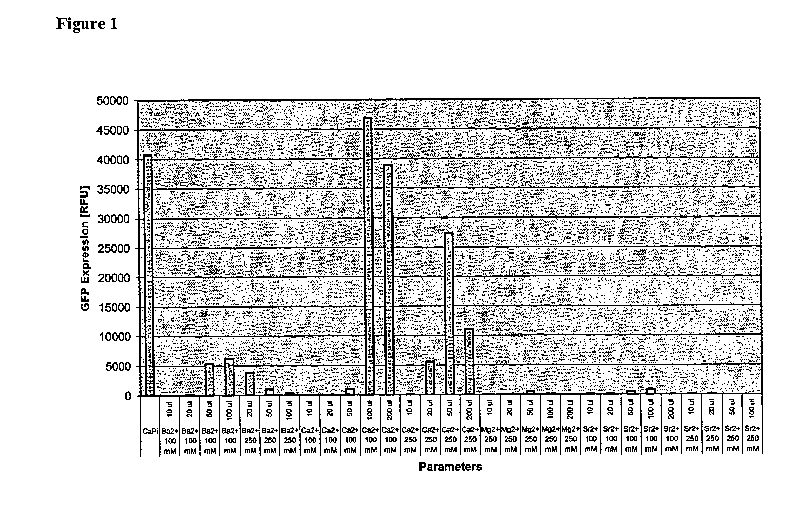

1 ml of a HEK 293 EBNA cell suspension per well was seeded into 12-well plates (TPP Trasadingen / Switzerland). The cells were seeded at a density of 500.000 cells/ml in FEME supplemented with 1% foetal calf serum (FCS) (JRH Biosciences Lenexa, KS). Two concentrations each, 100 mM and 250 mM, of Li⁺, Na⁺, K⁺, Cs⁺, Ca²⁺, Ba²⁺ and Sr²⁺ were prepared with ddH₂O. 25 µg of plasmid DNA encoding for GFP, pEGFP-N1 (Clontech), were mixed to 1 ml of each metal cation solutions. Five different addition volumes of metal cation solution were tested 10, 20, 50, 100 and 200 µl. Each of this volume for every metal cation and ion concentration was added to an individual well containing the cells. The experiment was performed in duplicates.
The mixture is incubated at 37°C with an atmosphere of 5% CO₂ on a orbital shaker, as described in Girard, P., M. Jordan, et al., 2001, Biochemical Engineering Journal 7(2): 117-119. Four hours post-transfection one volume of fresh prewarmed FEME supplemented with 2% FCS was added to the cell culture. The culture was further incubated at 37°C.
On day 3 after transfection GFP expression was measured. The cell were lysed by the addition of 1 ml of a PBS 1% Triton X-100 (EuroBio Les Ulis Cedex B, France) buffer to each well. After lysis for 60' the samples were measured in a Cytofluor® fluorescence plate (Applied Biosystems Foster City, CA) at an excitation wavelength of 485 nm and measured 530 nm. Results obtained for Ca²⁺, Ba²⁺ and Sr²⁺ are displayed in Figure 1. Results for GFP expression obtained for Li⁺, Na⁺, K⁺ and Cs⁺ were in the range of 50-100 RFU.

### B. Transfection of HEK 293 EBNA cells grown in suspension transfected in suspension with pEGFP-N1 under different conditions.

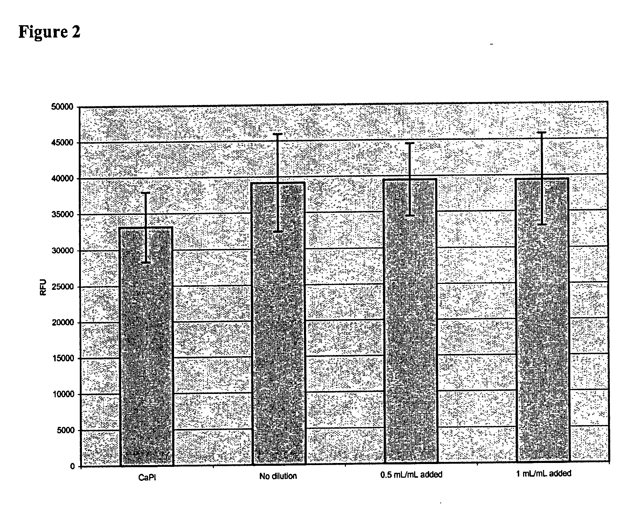

1 ml of a HEK 293 EBNA cell suspension per well was seeded into 12-well plates (TPP Trasadingen / Switzerland). The cells were seeded at a density of 500.000 cells/ml in FEME supplemented with 1% FCS (JRH Biosciences Lenexa, KS).
2.5 µg/ml of plasmid DNA encoding for GFP (pEGFP-N1 are mixed to 100 µl of 100 mM Ca²⁺ solution. DNA/Ca-solution is added to each individual well. The mixture is incubated on a rotational shaker in a CO₂ incubator. Four hours post-transfection different volumes of fresh prewarmed FEME supplemented with 1% FCS was added to the cell culture. The respective dilutions were without addition, half a volume and one volume of media added. The culture was further incubated at 37°C on a rotational shaker.
68 hours after transfection the cells in the wells are lysed with 1 ml of a PBS 1% Triton X-100 (EuroBio Les Ulis Cedex B, France) buffer. After lysis for 60' the samples were measured in a Cytofluor® fluorescence plate (Applied Biosystems Foster City, CA) at an excitation wavelength of 485 nm and measured 530 nm. Then fluorescence is measured. Results are displayed in Figure 2.

### C. Transfection of HEK 293 EBNA cells grown in suspension transfected in suspension and then left to adhere with pEGFP-N1.

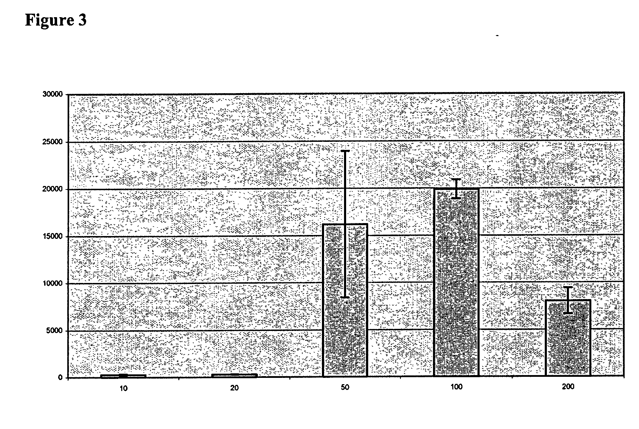

1 ml of a HEK 293 EBNA cell suspension per well was seeded into 12-well plates (TPP Trasadingen / Switzerland). The cells were seeded at a density of 200.000 cells/ml in FEME supplemented with 1% FCS (JRH Biosciences Lenexa, KS).
25 µg of plasmid DNA encoding for GFP (pEGFP-N1) are mixed to 1 ml of 100 mM Ca²⁺ solution. Different volumes of this solution, 10, 20, 50, 100 and 200 µl were added to each well. After addition/transfection cells are put back into the CO₂-incubator and left to adhere without agitation. Six hours post-transfection, 1 ml fresh, pre-warmed medium is added to each well. The culture is further incubated without agitation.
68 hours after transfection the cells in the wells are lysed with 1 ml of a PBS 1% Triton X-100 (EuroBio Les Ulis Cedex B, France) buffer. After lysis for 60' the samples were measured in a Cytofluor® fluorescence plate (Applied Biosystems Foster City, CA) at an excitation wavelength of 485 nm and measured 530 nm. Results are displayed in Figure 3.

### D.Transfection of HEK 293 EBNA cells grown in suspension transfected in suspension and then left to adhere with pEAX39LH1 and pEAX41LH2

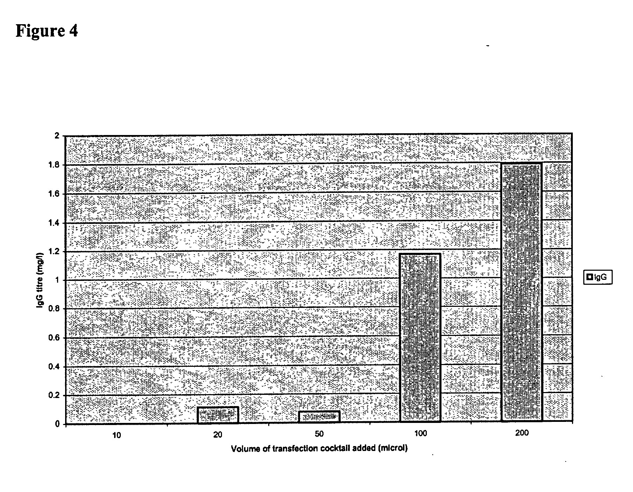

Transfection of HEK 293 EBNA cells grown in suspension transfected in suspension and then left to adhere with pEAX39LH1 and pEAX41LH2 has been carried out as described in 4.C. except that 2.5 µg/ml culture of the two plasmids pEAX39LH1 and pEAX41LH2 (30%:70%) were used. Results are displayed in Figure 4.

### E. Transfection of HEK 293 EBNA cells grown in adherence, transfected in suspension and then left to adhere with pEGFP-N1

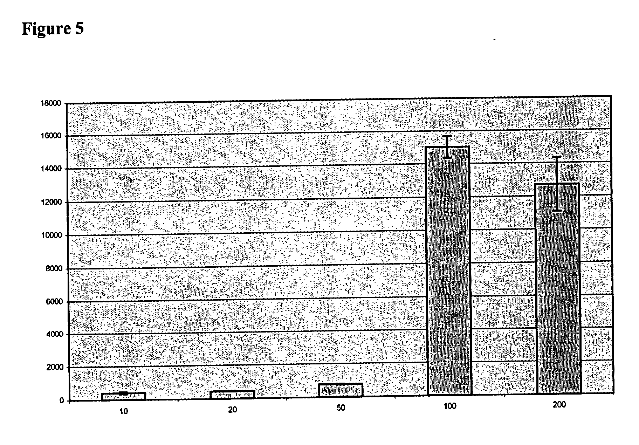

Adherent HEK 293 EBNA cell were detached using trypsin/EDTA (GibcoBRL, Paisley UK). The cell were resuspended in FEME supplemented with 1% FCS (JRH Biosciences Lenexa, KS) at a cells density of 200.000 cells/ml. 1 ml of suspension per well was seeded into 12-well plates (TPP Trasadingen / Switzerland). 25 µg of plasmid DNA encoding for GFP (pEGFP-N1) are mixed to 1 ml of 100 mM Ca²⁺ solution. Different volumes of this solution, 10, 20, 50, 100 and 200 µl were added to each well. After addition/transfection cells are put back into the CO₂-incubator and left to adhere without agitation.
Four to six hours post-transfection, the cell culture supernatant is discarded and 1 ml of fresh, prewarmed FEME with 2% FCS is added to each well. The cells were further incubated in a CO₂-incubator at 37°C without agitation.
68 hours after transfection the cells in the wells are lysed with 1 ml of a PBS 1% Triton X-100 (EuroBio Les Ulis Cedex B, France) buffer. After lysis for 60' the samples were measured in a Cytofluor® fluorescence plate (Applied Biosystems Foster City, CA) at an excitation wavelength of 485 nm and measured 530 nm. Results are displayed in Figure 5.

### F. Transfection of HEK 293 EBNA cells grown in adherence, transfected in suspension and then left to adhere with pEAX39LH1 and pEAX41LH2

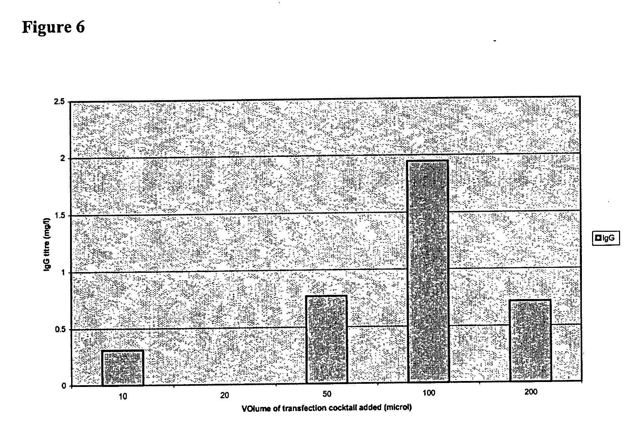

Transfection of HEK 293 EBNA cells grown in adherence, transfected in suspension and then left to adhere with pEAX39LH1 and pEAX41LH2 has been carried out as describe in 4.E.except that 2.5 µg/ml culture of the two plasmids pEAX39LH1 and pEAX41LH2 (30%:70%) were used. Results are displayed in Figure 6.

### G. GFP expression on the third day after transfection with the pEGFP-N1 vector at different pH.

1 ml of a HEK 293 EBNA cell suspension per well was seeded into 12-well plates (TPP Trasadingen / Switzerland). The cells were seeded at a density of 500.000 cells/ml in FEME without sodium carbonate, but supplemented with 1% FCS (JRH Biosciences Lenexa, KS). The media pH was adjusted by the addition of 1M HCl and 1 M NaOH (all Fluka) prior to the resuspension of the cells. 2.5 µg of plasmid DNA encoding for GFP, pEGFP-N1 (Clontech), were mixed to 100 µl of a 100 mM CaCl₂ solution, prepared with ddH₂O. The solution was then added to the wells containing the cell culture at the respective pHs.
The mixture is incubated at 37°C with an atmosphere of 5% CO₂ on a orbital shaker, as described earlier. Four hours post-transfection one volume of fresh prewarmed FEME supplemented with 1% FCS was added to the cell culture. The culture was further incubated at 37°C.
On day 3 after transfection GFP expression was measured. The cell were lysed by the addition of 1 ml of a PBS 1% Triton X-100 (EuroBio Les Ulis Cedex B, France) buffer to each well. After lysis for 60' the samples were measured in a Cytofluor® fluorescence plate (Applied Biosystems Foster City, CA) at an excitation wavelength of 485 nm and measured 530 nm. Very low expression is observed at pH lower than 7.5 (see Table 1). The expression peaks at 7.8 for the used Ca²⁺ concentrations. Surprisingly the cells survived well even at pH 8.

**Table 1:**

| GFP expression on the third day after transfection with the pEGFP-N1 vector at different pH. | | | | | | | | |
|---|---|---|---|---|---|---|---|---|
| **pH** | **6.5** | **7** | **7.2** | **7.4** | **7.5** | **7.6** | **7.8** | **8** |
| Expression (RFU) | 38.5 | -62.5 | 75 | 131.5 | 16954.5 | 15944 | 18302.5 | 12161 |

### H. Large-scale reactor run

In a 15 liter (nominal) stirred tank bioreactor (BioEngineering Wald, Switzerland) HEK 293 EBNA cells were seeded in 4000 ml FEME containing 2% FCS (JRH Biosciences Lenexa, KS) at a density of 300'000 cells/ml in to the reactor and incubated for 2 h at 37°C and pH 7.5. 5 mg plasmid DNA encoding for GFP (pEGFP-N1, Clontech Pablo Alto, CA) were mixed to 400 ml of an autoclaved 250 mM CaCl₂ (puriss. p.a.; Fluka Chemie AG Buchs, Switzerland) solution (ddH2O). The solution was immediately admixed to the cells. After an incubation period of 5 h the cell culture was extended to 8 liters by adding prewarmed FEME. The pH was only controlled after day one after transfection. It was set to 7.2. pH was controlled by adding NaOH 1M (Fluka) and CO₂ (Carba Gas Bern, Switzerland). Dissolved oxygen levels were maintained by continuous headspace sparing with filtered air. The culture was stopped on day 6 after transfection. GFP expression was measured offline. 1ml sample cell suspension from the reactor was lysed with 1 ml of a PBS 1% Triton X-100 (EuroBio Les Ulis Cedex B, France) buffer. After lysis for 60' the samples were measured in a Cytofluor® fluorescence plate (Applied Biosystems Foster City, CA) at an excitation wavelength of 485 nm and measured 530 nm.
The dO₂ levels did not fall beyond 30% of relative air saturation. The pH remained in the range of 7.0 to 7.2 during the pH controlled part of the run. Lactate levels reached 2.1 g/l and glucose fell to 1.7 g/l on the last day. Cell viability Cell density reached a maximum of 400'000 cells/ml on day 5 after transfection. More than 60% of the cells expressed GFP. The expression peaked on day 3 with above 16000 RFU and was then constant at 11000 RFU for the rest of the run.

### I. Admixing of DNA, Ca²⁺ and HEK 293 EBNA cells in different orders

1 ml HEK 293 EBNA cells, suspended in FEME medium supplemented with 1% fetal calf serum (JRH Biosciences Lenexa, KS), were seeded into each well of 12-well plates (TPP Trasadingen / Switzerland) at a density of 500.000 cells/ml. The cells were then incubated at 37°C at 100% air humidity for two hours. 2.5 µg plasmid DNA encoding the GFP, pEGFP-N1 vector (Clontech), and 100 µl of a 100 mM CaCl₂ prepared with ddH₂O were used to transfect each well. Transfection was done adding a solution of DNA and Ca²⁺ mixed in an Eppendorf tube to the cell culture as well as the two reagents separately and directly into the cell culture (once the Ca²⁺ before the DNA, once the DNA before the Ca²⁺). After transfection, the cells were reincubated for 96 hours at 37°C at 100% air humidity on an orbital shaker. Four hours post-transfection, fresh FEME medium supplemented with 1% fetal calf serum (JRH Biosciences Lenexa, KS) was added in a 1:1 ratio to the well plates, followed by further incubation under the same conditions as earlier. In order to assess transfection efficiency, cells are lysed after 96 hours using a PBS solution prepared with ddH₂O supplemented with 1% Triton X100 (EuroBio Les Ulis Cedex B, France). After a 1-hour incubation period under the same conditions as earlier, fluorescence is measured with Cytofluor (Applied Biosystems Foster City CA) using an excitation wavelength of 485 nm and measuring fluorescence at an emission wavelength of 530 nm. The same expression levels are obtained when adding the transfection reagents separately, and in random order, as when mixing DNA and CaCl₂-solution prior to addition.

### 5. Measuring expression

### A. Green Fluorescent Protein (GFP) production

The expression is measured through fluorescence emission. To measure the intensity of the fluorescence, the multiplate fluorescence reader CytoFluor® (Applied Biosystems Foster City, CA) is used. Fluorescence is read at 530 nm after excitation at 485 nm according to Chalfie et al., 1994 Science, 263, 802-5.

### B. Antibody production

The antibody concentration is measured with a sandwich ELISA method from a sample of the culture medium which ensures that only complete antibodies are detected according to Engvall and Perlman, 1971, Immuniochem., 8(9), 871-4.

### CaPi reference

As reference in the respective experiment cells were transfected according to the calcium phospate (CaPi) method (Jordan et al., Nucleic Acids Res., 1996 24(4), 596-601). For transfection, a 250 mM CaCl₂-solution as described under 2. is used together with a 1.4 mM PO₄³⁻-solution supplemented with 50 mM HEPES at a pH of 7.25. Two hours prior to precipitate formation cells are seeded at a cell density of 500.000/ml in FEME -medium supplemented with 1% fetal calf serum and incubated. 2.5µg DNA with 50µl Ca²⁺ and 50µl PO₄³⁻. After PO₄³⁻ addition, precipitate formation takes place for 1 minute followed by adding of 100µl to 1 ml culture. The mixture is incubated for 4 hours and 1 ml fresh, pre-warmed medium per ml culture is added. Expression was measured usually 68 hours after fresh, pre-warmed medium has been added.

### Conditioning/storage of DNA/metal cation solution

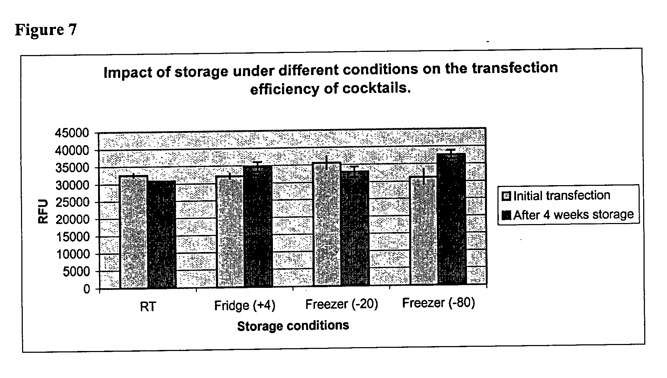

DNA and metal solution is prepared as described above in section 1 and 2. 100 µg DNA is added to 4 ml of Ca- solution. 800 µl of this DNA/Ca-solution was stored at room temperature, in a refrigerator or in a freezer (-20 to -80 °C) for 4 weeks. After 4 weeks frozen DNA was thawed and the Ca²⁺ and DNA containing solutions were directly applied to the cells. Cells were transfected prior to the storage as described before in experiment 4B with 100 µl of Ca²⁺/DNA solution. The experiment was identical to the one described in as experiment 4B, only limited to 100 ml transfection volume. After 4 weeks storage the experiments were repeated. The results (see Fig. 7) obtained indicate that the DNA/metal cation complex can be stored for at least 4 weeks without significant loss of transfection efficiency.

## Claims

1. A method for introducing a desired nucleic acid into a eukaryotic host cell, said method comprising
a) admixing the desired nucleic acid, a mono- and/or divalent metal cation and a eukaryotic host cell lacking a cell wall
b) incubating the mixture to allow the eukaryotic host cell to take up the nucleic acid.

2. The method of claim 1, wherein the desired nucleic acid is DNA.

3. The method of any of claim 1 or 2, wherein the concentration of the desired nucleic acid in the mixture is 0.1 to 10000 µg/ml.

4. The method of claim 1 to 3, wherein a divalent metal cation is used.

5. The method of claim 4, wherein the concentration of the divalent metal cation in the mixture is 0.001 mM to 2 M.

6. The method of claim 1 to 5, wherein the host cell is a mammalian cell.

7. The method of claim 1 to 6, wherein the host cell is a human cell.

8. The method of claim 1 to 7, wherein the mixture is incubated for a period of 0.5 to 24 hours.

9. A method of claim 1 to 8, wherein the mono- and/or divalent metal cation, the desired nucleic acid and the eukaryotic host cell lacking a cell wall are admixed in serum containing medium.

10. The method of claim 1 to 9, wherein the pH of the mixture is 5 to 9.

11. The method of claim 1 to 10, wherein the cells are separated from the mixture after the incubation step b) without further dilution of the mixture.

12. A method for storing a nucleic acid said method comprising
a) admixing a mono- and/or divalent metal cation and a desired nucleic acid
b) storing the solution of mono- and/or divalent metal cation and a desired nucleic acid for at least 8 weeks.

13. The method of claim 12, wherein a divalent metal cation is used.

14. The method of claim 13, wherein the concentration of the divalent metal cation in the solution is 10 mM to 10 M.

15. The method of claim 12 to 14, wherein the desired nucleic acid is DNA.
